# EUROPEAN PATENT APPLICATION

(11) **EP 1 830 291 A1**
(43) Date of publication of application: **05.09.2007**
(21) Application number: 07004281.7
(22) Date of filing: 01.03.2007
(51) Int. Cl.: G06F 19/00, H04L 12/28

(54) **Data management system, data transmitter-receiver, data management server, and method for data management**

(30) Priority: 02.03.2006 JP 2006056998
(71) Applicant: TANITA CORPORATION, Tokyo 174-8630 (JP)
(72) Inventor: Kosaka, Kazuhiro, Tokyo 174-8630 (JP)
(74) Representative: Müller-Boré & Partner Patentanwälte

(57) **Abstract**

A data management system or the like that can not add an extra load to an elderly person who is a user when measurement data is transmitted, can know parents' medical conditions in real time for a child, and can cut down expense comparatively.

A receiver 12 receives measurement data transmitted by a predetermined wireless communication system from the data measuring instrument 14 or the like, and transmits measurement information to a service provider side server 64. The service provider side server 64 records the measurement information onto DB 66 on the basis of a predetermined management scheme, and transmits a predetermined notification to a cellular phone 80 that has a predetermined relation to the measurement information. The cellular phone 80 transmits a transmission request inputted on the basis of the predetermined notification to the service provider side server 64, the service provider side server 64 transmits the contents recorded on the data base DB 66 on the basis of this transmission request to the cellular phone 80 under the predetermined condition, and the cellular phone 80 indicates the transmitted contents by the predetermined form.

## Description

### BACKGROUND OF THE INVENTION

### Field of the Invention

The present invention relates to a data management system or the like including a data transmitter-receiver, a data management server, and a mobile communication apparatus, which are mutually connected through a network.

### Description of the Related Art

A development of a health care system, collecting each measurement data of a user (target for measurement) measured by scales and a body fat monitor, or the like to a remote server and used for the health care, is advanced in recent years. In such a health care system, in general, each measurement data is input to a personal computer (PC) once, and transmitted to the server batch-processing afterwards. On the other hand, as aging society comes rapidly, the number of solitary old people homes in which they parts with their child and they lives alone respectively has grown. The development of the above-mentioned health care system increases in importance more and more in order to manage health in such an elderly person appropriately. (cf. "White Paper of Information and Communications in Japan, Year 2003", Chapter 1, Section 3, Ministry of Internal Affairs & Communications,<URL:http://www.johotsusintokei.soumu.go.jp/whitepaper/ja/ h15/pdf/F1030000.pdf>). It is similar to requiring nursing even if it is not an elderly person.

In the conventional health care system as the above-mentioned, it was necessary to input user's each measurement data to the PC once, and then to transmit it to the server batch-processing. Therefore, it was necessary to do a lot of operations to the elderly person or the person required nursing (hereinafter referred to as "Elderly person or the like") who is the user when the conventional health care system was applied to the solitary old people home or the home of the person required nursing or the like. The lots of operations were the operations of a locating of PC and necessary initialization first of all, the operations to input each measurement data to the PC continuously, and the operations to transmit it to the server batch-processing afterwards, or the like. However, there was a problem that doing the above-mentioned operation to the elderly person or the like who is the user put a strain significantly. Additionally, there was a problem of requiring expense not cheap for the purchase of the PC and the rental of the health care system, or the like. Moreover, it only knows parents' medical conditions in the batch even if the server is referred for the child of the elderly person or the like who is the user. Therefore, there was a problem that it was not able to know currently parents' medical conditions in real time.

### SUMMARY OF THE INVENTION

Then, the present invention has been achieved to solve the problems described above and it is an object of the present invention to provide a data management system or the like that can not add an extra load to the elderly person or the like who is the user in the solitary old people home or the like when the measurement data is transmitted, can know parents' medical conditions in real time for the child or the like of the elderly person or the like who is the user, and can cut down expense comparatively.

According to a first aspect of the present invention, there is provided a data management system comprising a data transmitter-receiver, a data management server and a mobile communication apparatus, which are mutually connected through a network, the data transmitter-receiver comprises: a measurement data receiving means for receiving measurement data transmitted from a data measuring instrument according to a predetermined wireless communication system; and a measurement information transmitting means for transmitting measurement information based on the measurement data received by the measurement data receiving means to the data management server, the data management server comprises: a measurement information processing means that records the measurement information transmitted by the measurement information transmitting means into a data recording unit on the basis of a predetermined management scheme, and transmits a predetermined notification to the mobile communication apparatus having a predetermined relation to the measurement information; and a recorded data transmitting means for transmitting contents recorded in the data recording unit under a predetermined condition to the mobile communication apparatus on the basis of requesting by the mobile communication apparatus, the mobile communication apparatus comprises: a request transmitting means for transmitting a request inputted on the basis of the predetermined notification transmitted by the measurement information processing means to the data management server; and a contents indicating means for indicating the contents transmitted by the recorded data transmitting means according to a predetermined form.

According to a second aspect of the present invention, there is provided a data transmitter-receiver connected with a data management server through a network, comprising: a measurement data receiving means for receiving measurement data transmitted from a data measuring instrument by a predetermined wireless communication system; and a measurement information transmitting means for transmitting measurement information based on the measurement data received by the measurement data receiving means to the data management server.

According to a third aspect of the present invention, there is provided a data management server connected with a data transmitter-receiver and a mobile communication apparatus through a network, comprising: a measurement information processing means that records measurement information based on measurement data transmitted from a data measuring instrument to the data transmitter-receiver according to a predetermined wireless communication system and transmitted from the data transmitter-receiver, on the basis of a predetermined management scheme in a data recording unit, and transmits a predetermined notification to the mobile communication apparatus having a predetermined relation to the measurement information; and a recorded data transmitting means for transmitting contents recorded in the data recording unit under a predetermined condition to the mobile communication apparatus on the basis of a request by the mobile communication apparatus.

According to a fourth aspect of the present invention, there is provided a method for data management used by a data transmitter-receiver, a data management server and a mobile communication apparatus which are mutually connected through a network, comprising: a measurement data receiving step at which the data transmitter-receiver receives measurement data transmitted from a data measuring instrument according to a predetermined wireless communication system; a measurement information transmitting step at which the data transmitter-receiver transmits measurement information based on the measurement data received in the measurement data receiving step to the data management server; a measurement information processing step at which the data management server records the measurement information transmitted in the measurement information transmitting step in a data recording unit on the basis of a predetermined management scheme, and transmits a predetermined notification to the mobile communication apparatus having a predetermined relation to the measurement information; a request transmitting step at which the mobile communication apparatus transmits a request inputted on the basis of the predetermined notification transmitted in the measurement information processing step to the data management server; a recorded data transmitting step at which the data management server transmits the contents recorded in the data recording unit to the mobile communication apparatus under a predetermined condition, on the basis of the request transmitted in the request transmitting step; and a contents display step at which the mobile communication apparatus indicates the contents transmitted in the recorded data transmitting step by a predetermined form.

The above and other objects, effects, features and advantages of the present invention will become more apparent from the following description of the embodiments thereof taken in conjunction with the accompanying drawings.

### BRIEF DESCRIPTION OF THE DRAWINGS

Fig. 1 shows a data management system 1 according to a first embodiment of the present invention.
Fig. 2 shows a detailed configuring of the home system 10.
Fig. 3 shows configuring of the receiver 12.
Fig. 4 shows the installation method of specific small power transmitting module MD.
Fig. 5 shows the flow of the method of the data management according to the embodiment of the present invention.
Fig. 6 shows a data management system 2 according to a second embodiment of the present invention.
Fig. 7 shows a data management system 3 according to a third embodiment of the present invention.

### DETAILED DESCRIPTION OF THE PREFERRED EMBODIMENTS

Various embodiments of the present invention will be described herein below with reference to the accompanying drawings. It is to be noted that the same or similar reference numerals are applied to the same or similar parts and elements throughout the drawings, and the description of the same or similar parts and elements will be omitted or simplified.

### First Embodiment

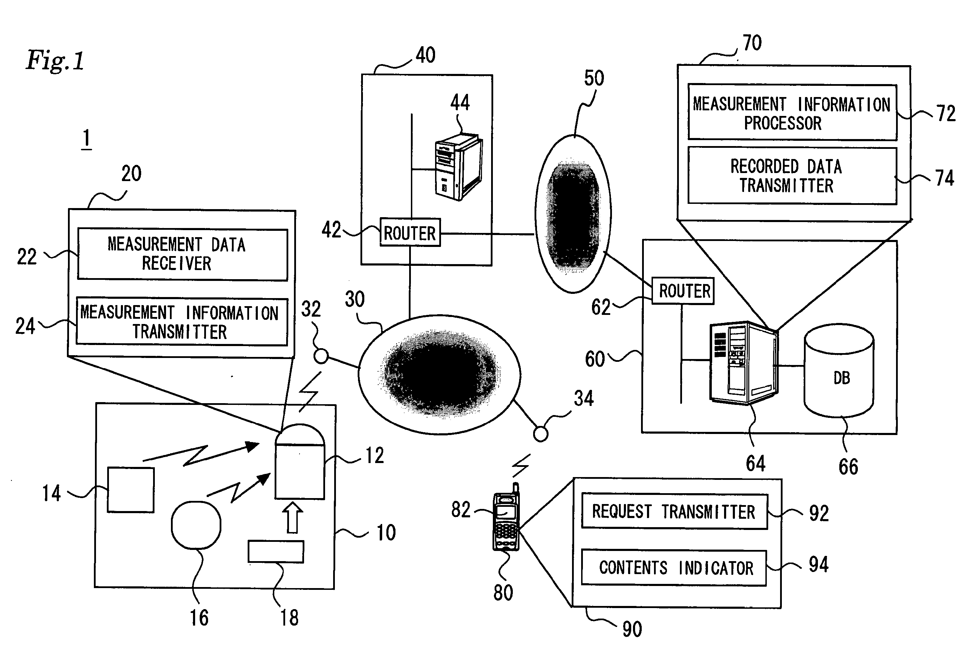

Fig. 1 shows a data management system 1 according to a first embodiment of the present invention. In Fig. 1, reference numeral 30 is a cellular phone (or mobile phone) communication network; 32 and 34 are base stations provided for the cellular phone communication network 30; 10 is a home system at especially solitary old people home or the like; 12 is a receiver (data transmitter-receiver) set up in the home system 10, and connected to the cellular phone communication network 30 via base station 32; 40 is a cellular phone carrier side system; 44 is a cellular phone carrier (a telephone carrier) side server connected to the cellular phone communication network 30 via a router 42; reference numeral 50 is a dedicated line network of Integrated Services Digital Network (ISDN) or Internet Protocol-Virtual Private Network (IP-VPN), or the like; 60 is a system on health care service provider (hereafter referred to as a "service provider") side where the health care service or the like are done; 64 is a service provider side server (data management server) connected to the dedicated line network 50 via a router 62; 66 is a data base DB (data recording unit) recording data for the health care of the user of the home system 10; and 80 is a cellular phone (mobile communication apparatus) connected with the cellular phone communication network 30 through the base station 34. As shown in Fig. 1, the receiver 12, the service provider side server 64, and the cellular phone 80 are mutually connected through the cellular phone communication network 30 and the dedicated line network 50.

Next, the receiver 12 will be explained. In Fig. 1, reference numeral 20 is a functional block showing the function of the receiver 12. As shown in Fig. 1, the receiver 12 comprises a measurement data receiver (measurement data receiving means) 22 for receiving the measurement data transmitted from data measuring instruments 14, 16 and 18 or the like according to the predetermined wireless communication system and measurement information transmitter (measurement information transmitting means) 24 for transmitting measurement information based on the measurement data received from the measurement data receiver 22 to the service provider side server 64. The data measuring instrument 14 or the like and the predetermined wireless communication system will be described later.

In Fig. 1, reference numeral 70 is a functional block showing the function of the service provider side server 64. A measurement information processor (measurement information processing means) 72 of the service provider side server 64 records the measurement information transmitted from the measurement information transmitter 24 onto the data base DB 66 on the basis of the predetermined management scheme. Various management scheme for doing the system construction by the purposes of a vital data storage, the system control management, and the service conduct, or the like is preferable as the predetermined management scheme. Furthermore, the measurement information processor 72 transmits the predetermined notification to the cellular phone 80 that has the predetermined relation to the measurement information. The user in home system 10 is especially assuming the elderly person or the person required nursing (Hereinafter referred to as "Elderly person or the like"). Therefore, the cellular phone that the child or the relative, or the like in this elderly person or the like use is preferable as the cellular phone 80 that has the predetermined relation to the measurement information of the elderly person or the like. However, it is a surely such as they may be Personal Handyphone System (PHS), and be other mobile information processing terminal equipments, since the cellular phone 80 is one example of the mobile communication apparatus. Even if it is not a child or it is not a relative or the like in this elderly person or the like, it is acceptable as the person where the person in the standpoint who manages health or the like of this elderly person or the like, such as a home helper, a care manager or the like, also has the predetermined relation to the measurement information of this elderly person or the like. In the following, the person who has above-mentioned predetermined relation is referred to as "Child or the like". The predetermined notification is a notification to the effect that measurement information is recorded or the like. It is preferable that this notification is transmitted in the form of the Electronic Mail or the like via the dedicated line network 50 and the cellular phone communication network 30. The service provider side server 64 comprises a recorded data transmitter 74 (recorded data transmitting means) for transmitting the contents of measurement information or the like recorded in the data base DB 66 on the basis of requesting from cellular phone 80 to the cellular phone 80 under the predetermined condition. The input of just user ID and a password is preferable as a proof of being those who have the above-mentioned predetermined relation as the predetermined condition.

In Fig. 1, reference numeral 90 is a functional block showing the function of the cellular phone 80. The cellular phone 80 comprises a request transmitter (request transmitting means) 92 for transmitting a transmission request or the like of measurement information input by the person who has above-mentioned predetermined relation to the service provider side server 64 on the basis of the predetermined notification transmitted by the measurement information processor 72. Since the predetermined notification is transmitted in the form of the Electronic Mail or the like as the above-mentioned, Uniform Resource Locator (URL) of the home page or the like of the transmitting side (service provider side server 64) is shown in this notification. Therefore, a user may access this URL and to input the user ID and the password. The cellular phone 80 comprises a contents indicator (contents indicating means) 94 for indicating the contents of the measurement information or the like transmitted from the recorded data transmitter 74 according to the predetermined form. Although it is preferable that it is the form which displays the contents, such as measurement information, on the display 82 of the cellular phone 80 as the predetermined form, of course, it may be the form by voice guidance. Moreover, it may use the form by the audio guidance together with the display type to display 82.

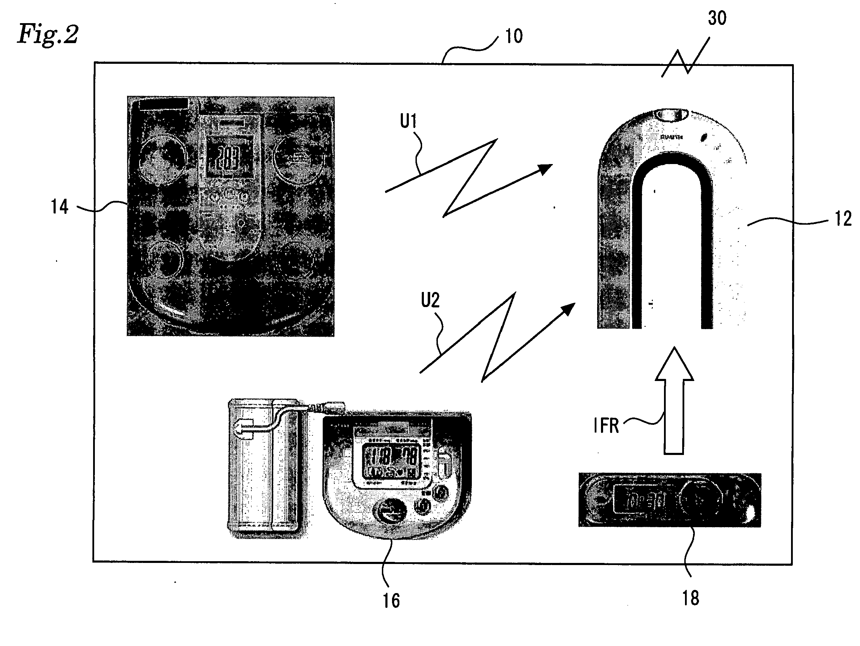

Fig. 2 shows a detailed configuring of the home system 10. The same reference numerals in Fig. 2 as those in Fig. 1 denote the same elements, and therefore explanations thereof will be omitted. The receiver 12 is shown in Fig. 2 for the convenience of the drawing by the same size as other data measuring instruments 14 or the like. However, in practice, it is the compact size about a notebook and it is possible after purchase to begin to use immediately only by taking out from the packed-up box and switching on a power supply. Therefore, the operation or the like of a locating of the PC and required initialization like conventional is unnecessary. As shown in Fig. 2, the data measuring instrument 14 is the body composition monitors of the scales and body fat monitor or the like, the data measuring instruments 16 is an electronic sphygmomanometer, and the data measuring instrument 18 is a pedometer. It is preferable that data measuring instrument 14 or the like are measuring instruments of the data that relates to the living body in the elderly person or the like who is the target for measurement. It may be a urine glucose meter, and be a clinical thermometer, or the like excluding above-mentioned data measuring instrument 14 or the like. Although three data measuring instruments 14 or the like are shown in Fig. 2, this is convenience in the drawing, the number of data measuring instruments 14 or the like is not limited to three, and the data measuring instrument of the desired number can be set up. The communication system (predetermined wireless communication system) of between the data measuring instrument 14, 16 and the receiver 12 can be assumed to be a specified low power radio. The specified low power radio is a radio that does not require the license, and it is a radio for the short distance of 10 mW or less of the antenna power (radio equipment standard ARIBSTD-T67 for the specified low power radio station telemeter, for the telecontrol, and for the data transmission). It is possible to communicate even in the situation without the prospect although the distance that can be communicated is about 50 m to 100 m. The communication system (predetermined wireless communication system) of between the data measuring instrument 18 and the receiver 12 can be achieved by infrared rays. It may use a format of general Association for Electric Home Appliances or an original format, or the like as a format of infrared transmitting. The measurement data can be automatically transmitted to the receiver 12 by an easy operation such as providing the data measuring instrument 18 on the receiver 12. The measurement data can be transmitted to the receiver 12 automatically and in real time by using the above-mentioned communication system. Therefore, an extra load can be never added to the elderly person or the like when the measurement data is transmitted, and the child or the like of the elderly person or the like who is the user can know the condition of the parents' health in real-time.

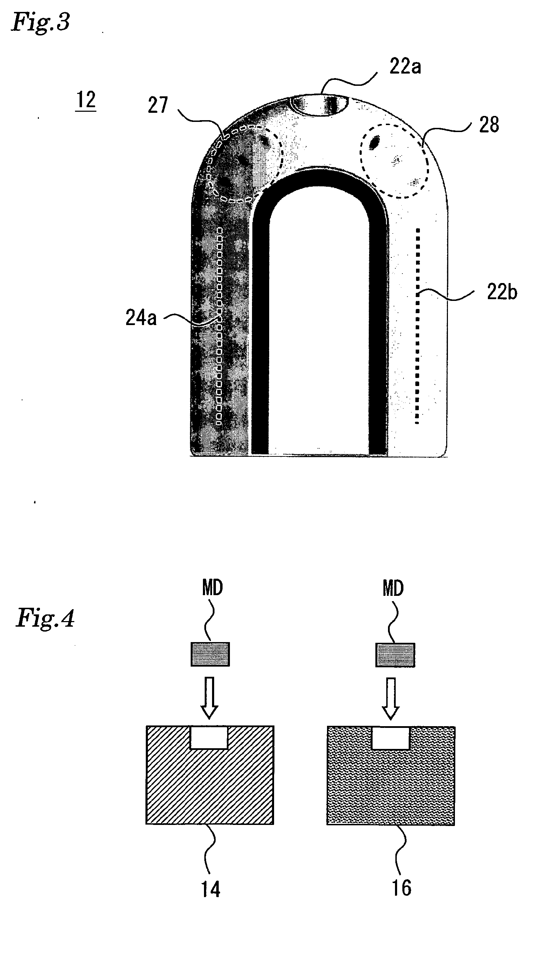

Fig. 3 shows configuring of the receiver 12. In Fig. 3, reference numeral 22a is an infrared receiver that is an example of the measurement data receiver 22; 22b is an antenna for specified low power radio that is an example of the measurement data receiver 22; and 24a is an antenna for cellular phone that is an example of the measurement information transmitter 24. It may use both parts usually used. Since both of the antenna for specified low power radio 22b and the antenna for cellular phone 24a are built into the receiver 12, it is shown in the dotted line in Fig. 3. Reference numeral 27 is an antenna intensity (or a field intensity) indicator for indicating antenna intensity of the antenna for cellular phone 24a; and 28 is a communication status indicator for indicating the communication status of the cellular phone. The antenna intensity indicator 27 and the communication status indicator 28 may be configured by using LED (Light Emitting Diode). The communication operating sound can be output by a voice output unit (not show in the figure), and the communication situation and the malfunction or the usage or the like of data measuring instrument 14 or the like can be output by the audio guidance.

A plurality of personal number buttons (not shown) are provided on the body composition monitors 14 or the like. The elderly person or the like who is the user turns on the power supply of receiver 12, and pushes his or her own number of the personal number button of the body composition monitors 14 or the like, and measures. Thereby, the receiver 12 can certify automatically the elderly person or the like who is the user. The receiver 12 receives the measurement data transmitted from the body composition monitors 14 and the sphygmomanometer 16 by the specified low power radio by the antenna for specified low power radio 22b, and receives the measurement data transmitted from the pedometer 18 by infrared rays by the infrared receiver 22a. The received measurement data is transmitted to the base station 32 via the antenna for cellular phone 24a as the measurement information including ID of receiver 12 or the like. Therefore, the receiver 12 can be set up anywhere in home system 10 within the wireless radius of the electric wave of the cellular phone. The measurement information transmitted to the base station 32 is recorded in the data base DB 66 by the service provider side server 64 through the cellular phone communication network 30 and the dedicated line network 50. That is, the measurement data that was measured by the data measuring instrument 14 or the like and received with the receiver 12 is transmitted to the service provider side server 64 as the measurement information automatically and in real time. Since it is also possible to pay the charge of the cellular phone on the service provider side, the defrayal in the elderly person or the like who is the user can be reduced. The receiver 12 can obtain time data from the service provider side server 64, and can do the time setting to the timer automatically by the obtained time data.

Fig. 4 shows the installation method of specified low power transmitting module MD. The same reference numerals in Fig. 4 as those in Fig. 1 denote the same elements, and therefore explanations thereof will be omitted. As shown in Fig. 4, the same specified low power transmitting module MD for specified low power transmitting can be embedded to the body composition monitors 14 and the sphygmomanometer 16, or the like. Therefore, a conventional data measuring instrument not comprising communication function can be used simply and at a low price in the home system 10 by embedding the same specified low power transmitting module MD in various data measuring instruments 14 or the like.

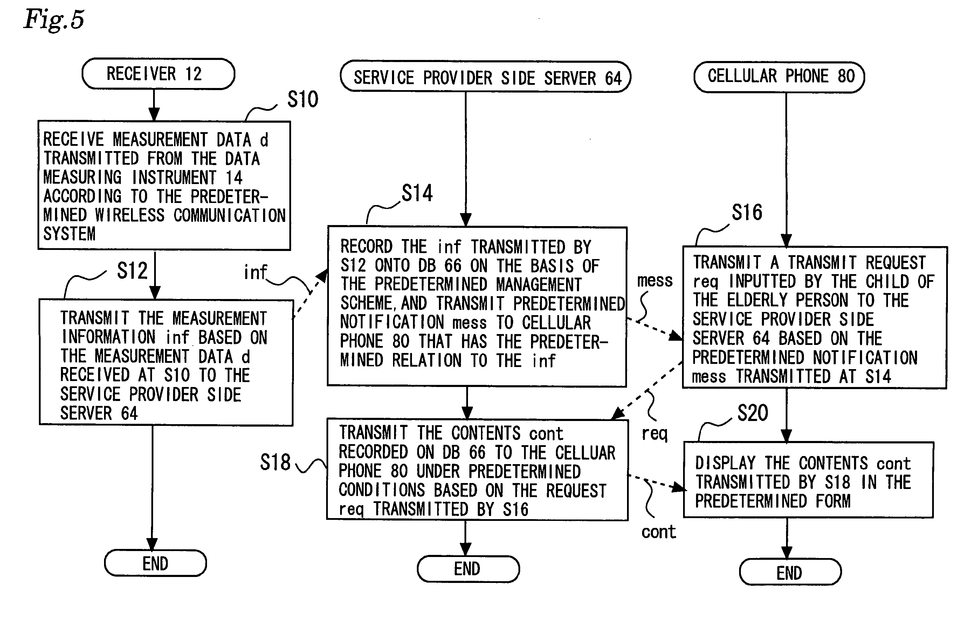

Fig. 5 shows the flow of the method for the data management according to the present invention as flowchart. In Fig. 5, a left flowchart shows the flow of the processing of receiver 12 sides, the flowchart at the center shows the flow of the processing of service provider side server 64, a right flowchart shows the flow of the processing of cellular phone 80 sides, and the communication of each other is shown by the dotted line that connects with each flowchart. As shown in Fig. 5, first of all, the receiver 12 receives measurement data d transmitted from the data measuring instrument 14 or the like according to the predetermined wireless communication system (measurement data receiving step: step S10). Next, the receiver 12 transmits the measurement information inf based on the measurement data d received at the measurement data receiving step (Step S10) to the service provider side server 64 (measurement information transmitting step: Step S12).

The service provider side server 64 continuously records the measurement information inf transmitted by the measurement information transmitting step (step S12) onto the data base DB 66 on the basis of the predetermined management scheme, and transmits predetermined notification mess to cellular phone 80 that has the predetermined relation to the measurement information inf (measurement information processing step: step S14).

The cellular phone 80 transmits a transmission request req inputted by the child or the like of the elderly person or the like to the service provider side server 64 based on the predetermined notification mess transmitted at the measurement information processing step (Step S14) (request transmitting step: Step S16).

The service provider side server 64 transmits the contents cont recorded on the database DB 66 to the cellular phone 80 under predetermined conditions based on the request req transmitted by the request transmitting step (Step S16) (record data transmitting step: Step S18).

The cellular phone 80 displays the contents cont transmitted by the record data transmitting step (Step S18) in the predetermined form (contents display step: Step S20). In addition, in the above-mentioned embodiment, the predetermined notification mess is transmitted from the service provider side server 64 to the cellular phone 80 in real time at each measurement by the data measuring instrument 14 or the like. However, it is also possible to transmit the predetermined notification mess by setting up specified time from the cellular phone 80 to the service provider side server 64 at the specified time. Furthermore, it is also possible by requesting the predetermined notification mess of the service provider side server 64 from the cellular phone 80 to display the contents cont at arbitrary time. By these, those who have time restrictions like an office worker or the like can also use this data management system 1 (the below-mentioned data management system 2 and 3 or the like are also included) in comfort.

As mentioned above, according to the first embodiment of the present invention, the data management system1 comprises the receiver 12, the service provider side server 64, and the cellular phone 80 mutually connected through the cellular-phone communications network 30 and the dedicated line network 50. The receiver 12 comprises the measurement data receiver 22 that receives measurement data of the elderly person or the like transmitted according to the predetermined wireless communication system from the data measuring instrument 14, 16 and 18, and the measurement information transmitter 24 that transmits the measurement information based on the measurement data received by the measurement data receiver 22 to the service provider side server 64. The measurement information processor 72 of the service provider side server 64 records the measurement information transmitted from the measurement information transmitter 24 onto the data base DB 66 on the basis of the predetermined management scheme, and transmits the predetermined notification to the cellular phone 80 that has the predetermined relation to the measurement information. The cellular phone 80 comprises the request information transmitter 92 that transmits a transmission request or the like of measurement information inputted by those who have the above-mentioned predetermined relation to the service entrepreneur side server 64 based on the predetermined notice transmitted by the measurement information processor 72. The service provider side server 64 comprises the recorded data transmitter 74 for transmitting the contents of measurement information or the like recorded on the data base DB 66 to the cellular phone 80 under the predetermined condition on the basis of requesting from the cellular phone 80. The cellular phone 80 comprises the contents indicator 94 for indicating the contents of measurement information or the like transmitted by the recorded data transmitter 74 by the predetermined form.

After purchase, it takes out from the packed-up box, a power supply is only switched on, and the receiver 12 can begin to be used immediately. Therefore, the conventional operations of a locating of the PC and necessary initialization or the like are unnecessary. It is preferable that data measuring instruments 14 or the like are the measuring instruments of the data that relates to the living body in the elderly person or the like who is the target for measurement. The predetermined wireless communication system can be made the specified low power radio and infrared rays, or the like according to the data measuring instrument 14 or the like. The measurement data can be transmitted to the receiver 12 automatically and in real time by using the above-mentioned communication system. Therefore, never adding an extra load to the elderly person or the like when the measurement data is transmitted, and knowing parents' health conditions in real time for the child or the like of the elderly person or the like who is the user becomes possible. The measurement data received with the receiver 12 is transmitted to the base station 32 as measurement information through the cellular phone communication network 30 and the dedicated line network 50, and recorded in the data base DB 66 by the service provider side server 64. That is, the measurement data that was measured by the data measuring instrument 14 or the like and received with the receiver 12 is transmitted to the service provider side server 64 automatically and in real time. It is also possible to pay the charge of the cellular phone on the service provider side. Therefore, there is an effect that the defrayal in the elderly person or the like who are the users can be reduced, and expense can be cut down to low comparatively. In addition, it is necessary to input living body information on the height, the ages, and sexes, or the like of the elderly person or the like who is the user to the body composition monitors 14. However, it is also possible to input to the data base DB 66 by using cellular phone 80 or another means instead of inputting directly to the body composition monitors 14, and to transmit the information to the body composition monitors 14 through the cellular phone network 30 and the receiver 12. Therefore, in this case, the load of the elderly person or the like who is the user can be reduced.

### Second Embodiment

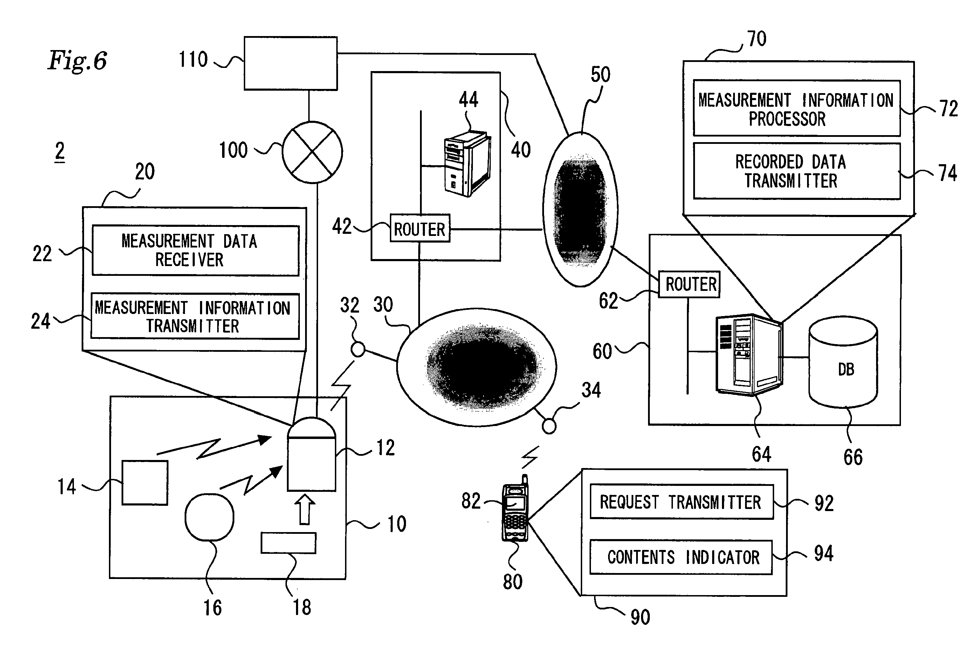

Fig. 6 shows a data management system 2 according to a second embodiment of the present invention. The same reference numerals in Fig. 6 as those in Fig. 1 denote the same elements, and therefore explanations thereof will be omitted. In Fig. 6, reference numeral 100 is a fixed-line phone network, and reference numeral 110 is a fixed-line phone carrier side system. As shown in Fig. 6, it can be the connected by the fixed-line phone network 100 between the receiver 12 and the fixed-line phone carrier side system 110. In this case, it may provide a terminal (not show) for the wire connection for the receiver 12. It is possible to set it up in the arbitrary location if there is a fixed-line phone line even if receiver 12 is a location without the wireless radius of the electric wave of cellular phone. It connects with the dedicated line network as well as the first embodiment between the fixed-line phone carrier side system 110 and the service provider side server 64. The number of the base stations 32 or the like of cellular phone network 30 is significantly little in the mountains area or the like compared with the urban area. Therefore, appropriate health care service can be provided for the elderly person or the like who resides in the depopulated area by using the data management system 2. It is also possible that the user selects the fixed-line phone network 100 or the cellular phone network 30 considering the fee and the usage, or the like arbitrarily.

As mentioned above, according to the second embodiment of the present invention, it is possible to connect it by the fixed-line phone network 100 between the receiver 12 and the fixed-line phone carrier side system 110. Appropriate health care service can be provided for the elderly person or the like who resides in the depopulated area by using data management system 2. It is also possible that the user selects the fixed-line phone network 100 or the cellular phone network 30 considering the fee or the like arbitrarily. Therefore, in addition to the effect of the first embodiment, the defrayal in the elderly person or the like who is the user can be furthermore reduced, and expense can be cut down to low.

### Third Embodiment

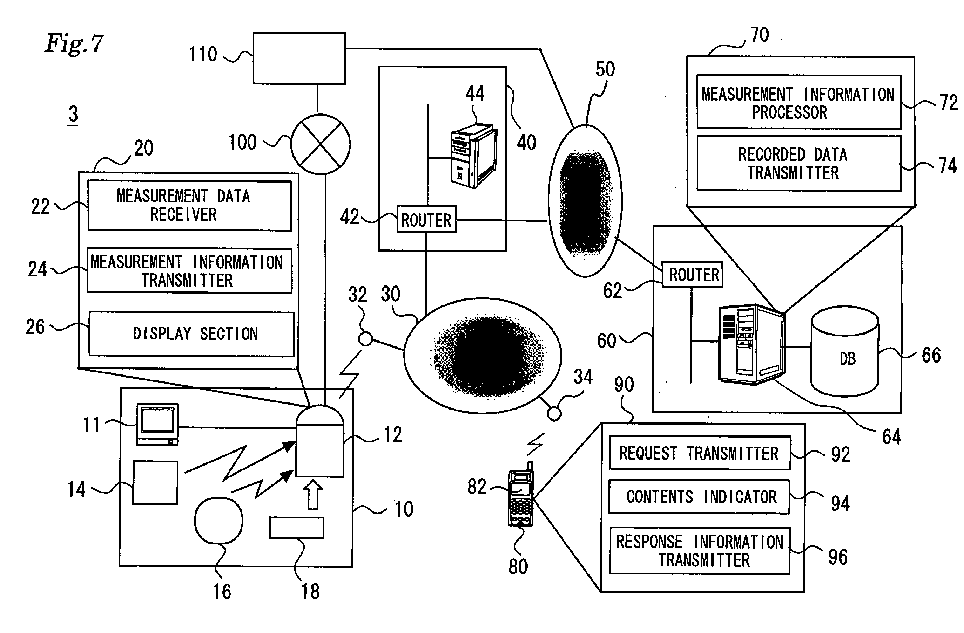

Fig. 7 shows a data management system 3 according to a third embodiment of the present invention. The same reference numerals in Fig. 7 as those in Fig. 6 denote the same elements, and therefore explanations thereof will be omitted. In Fig. 7, reference numeral 96 is a function added to the cellular phone 80, and is a response information transmitter (response information transmitting means) for transmitting response information based on the contents indicated from the contents indicator 94 to receiver 12. The elderly person or the like who is the user and the child or the like can transmit a desired answer to the receiver 12 in the home system 10 after seeing (and/or hearing) the contents indicated by the contents indicator 94 through the base station 34, the cellular phone network 30, and the base station 32 by using the Electronic Mail. As shown in Fig. 7, a display (display unit) 11 can be furthermore connected with the receiver 12, and the response information transmitted from the response information transmitter 96 can be displayed on the display 11 by the function of display section (display means) 26.

As mentioned above, according to the third embodiment of the present invention, the display 11 can be furthermore connected with the receiver 12. The elderly person or the like who is the user and the child or the like can transmit a desired answer to the receiver 12 in the home system 10 after seeing (and/or hearing) the contents indicated by the contents indicator 94 through the base station 34, or the like by using the Electronic Mail. The display section 26 can display the response information transmitted by the response information transmitter 96 on display 11. As a result, in addition to the effect of the embodiment mentioned above, the elderly person or the like who is the user can simply recognize that the child or the like learnt his medical condition.

### Fourth Embodiment

In the embodiments mentioned above, it is assumed that the data measuring instrument 14 or the like are measuring instruments of the data that related to the living body in the elderly person or the like who is the target for measurement. However, it may be a measuring instrument of the data that relates to the environment of the target for measurement as the data measuring instrument. For example, an illumination switch that detects the change in brightness can be used as a data measuring instrument for elderly person or the like who is the user for entering a room in home system 10 and turning on lighting. As a result, the fact of having turned on lighting is transmitted to the cellular phone 80 through the service provider side server 64 from the receiver 12 on real time. Therefore, the child or the like of the elderly person or the like who is the user can know the fact that this elderly person or the like has entered a room, that is, the change in the environment in this elderly person or the like at once. When the lighting is turned off, the aforementioned fact is similarly transmitted to cellular phone 80 at once. Therefore, the child or the like can know the change in the environment in this elderly person or the like of having a the room (or went to bed) at once.

As a measuring instrument of the data that relates to the environment of the target for measurement, it is realizable with a door opening and closing switch. By installing the door opening and closing switch on the door or the window, or the like in the home system 10, whenever the elderly person or the like who is the user opens and closes the door or the window, or the like, this fact is in real time transmitted from the receiver 12 to the cellular phone 80 through the service provider side server 64. Therefore, the child or the like can know the change of the environment of this elderly person or the like immediately.

As a measuring instrument of the data that relates to the environment of the target for measurement, it is realizable with an infrared sensor. By installing the infrared sensor on the desired position in the home system 10, whenever the elderly person or the like who is the user is detected in the infrared sensor, this fact is in real time transmitted from the receiver 12 to the cellular phone 80 through the service provider side server 64. Therefore, the child or the like can know the change of the environment of this elderly person or the like immediately.

As the data measuring instrument, it may be a measuring instrument of the data that relates to the situation of the target for measurement. It is realizable with an emergency report button as a measuring instrument of the data. The emergency report button may be installed on the desired position in the home system 10, or it may attach to the body of the elderly person or the like who is the user as the pendant or the like. As a result, whenever the elderly person or the like pushes the emergency report button, this fact is in real time transmitted from the receiver 12 to the cellular phone 80 through the service provider side server 64. Therefore, the child or the like can know immediately change (state of emergency or the like) of the situation of this elderly person or the like.

As mentioned above, according to the fourth embodiment of the present invention, it can also consider as the measuring instrument of the data relevant to the environment and/or the situation for the target for measurement as a data measuring instrument. As a data measuring instrument, it can constitute at least one of the measuring instrument of the data relevant to living bodies of the elderly person or the like for the target for measurement, the measuring instrument of the data relevant to the environment for the target for measurement, and the measuring instrument of the data relevant to the situation for the target for measurement. As a result, the child or the like can easily conprovider safety in the elderly person or the like.

### Industrial Applicability

The present invention can be especially applied to the health care system intended for the elderly person or the like in the solitary old people home.

According to the data management system or the like of the present invention, a receiver, a service provider side server and a cellular phone are mutually connected through a cellular phone communication network and a dedicated line network. The receiver includes a measurement data receiver and a measurement information transmitter. The measurement data receiver especially receives measurement data of the elderly person or the like transmitted according to a predetermined wireless communication system from a data measuring instrument. The measurement information transmitter transmits measurement information based on the measurement data received by the measurement data receiver to a service provider side server. The measurement information processor of the health care service provider side server records the measurement information transmitted by the measurement information transmitter onto a data base DB on the basis of the predetermined management scheme, and transmits the predetermined notification to the cellular phone that has the predetermined relation to the measurement information. The cellular phone comprises a request information transmitter. The request information transmitter transmits a transmission request or the like of measurement information inputted by those who have the above-mentioned predetermined relation to the health care service provider side server based on the predetermined notice transmitted by the measurement information processor. The health care service provider side server comprises a recorded data transmitter for transmitting the contents of measurement information or the like recorded on the data base DB to the cellular phone under the predetermined condition on the basis of requesting from the cellular phone. The cellular phone comprises a contents indicator for indicating the contents of measurement information or the like transmitted by the recorded data transmitter according to the predetermined form.

It is possible after purchase to begin to use immediately only by taking out from the packed-up box and switching on a power supply. Therefore, the operation or the like of a locating of the PC and required initialization like conventional is unnecessary. It is preferable that data measuring instruments or the like are the measuring instruments of the data that relates to the living body in the elderly person or the like who is the target for measurement or other measuring instruments. The predetermined wireless communication system can be made the specified low power radio and infrared rays, or the like according to the data measuring instrument. The measurement data can be transmitted to the receiver automatically and in real time according to the above-mentioned communication system. Therefore, never adding an extra load to the elderly person or the like when the measurement data is transmitted, and knowing parents' medical conditions in real time for the child or the like of the elderly person or the like who is the user become possible. The measurement data received with the receiver is transmitted to the base station as measurement information through the cellular phone communication network and the dedicated line network, and recorded in the data base DB by the health care service provider side server. That is, the measurement data that was measured by the data measuring instrument and received with the receiver is transmitted to the health care service provider side server automatically and in real time. It is also possible to pay the charge of the cellular phone on the health care service provider side. Therefore, there is an effect that the defrayal in the elderly person or the like who is the user can be reduced, and expense can be cut down to low comparatively.

In the data management system, the network between the data transmitter-receiver and a telephone carrier side system may be a cellular phone communication network or a fixed-line phone network, and the network between the telephone carrier side system and the data management server may be a dedicated line network.

Here, the mobile communication apparatus may further comprise a response information transmitting means for transmitting response information based on the contents indicated by the contents indicating means to the data transmitter-receiver, the data transmitter-receiver may further comprise a display unit and a display means for displaying the response information transmitted by the response information transmitting means on the display unit.

In the data management system, the data measuring instrument may be at least one of a measuring instrument of data that relates to a living body of a target for measurement, a measuring instrument of data that relates to an environment of a target for measurement and a measuring instrument of data that relates to a situation of a target for measurement.

In the data transmitter-receiver, the network between the data transmitter-receiver and a telephone carrier side system may be a cellular phone communication network or a fixed-line phone network, and the network between the telephone carrier side system and the data management server is a dedicated line network.

In the data transmitter-receiver, the measurement information transmitted by the measurement information transmitting means may be recorded in a data recording unit by the data management server on the basis of a predetermined management scheme, and a predetermined notification may be transmitted to a mobile communication apparatus having a predetermined relation to the measurement information, the mobile communication apparatus connected through the network, a request inputted to the mobile communication apparatus side on the basis of the predetermined notification may be transmitted to the data management server by the mobile communication apparatus, contents recorded in the data recording unit may be transmitted to the mobile communication apparatus by the data management server under a predetermined condition according to the request, the contents may be indicated by using a predetermined form by the mobile communication apparatus, and response information based on the indicated contents may be transmitted to the data transmitter-receiver, the response information may be displayed on a display unit of the data transmitter-receiver side.

In the data transmitter-receiver, the data measuring instrument may be at least one of a measuring instrument of data that relates to a living body of a target for measurement, a measuring instrument of data that relates to an environment of a target for measurement and a measuring instrument of data that relates to a situation of a target for measurement.

In the data management server, the network between the data transmitter-receiver and a telephone carrier side system may be a cellular phone communication network or a fixed-line phone network, and the network between the telephone carrier side system and the data management server may be a dedicated line network.

In the data management server, the data measuring instrument may be at least one of a measuring instrument of data that relates to a living body of a target for measurement, a measuring instrument of data that relates to an environment of a target for measurement, and a measuring instrument of data that relates to a situation of a target for measurement.

The present invention has been described in detail with respect to various embodiments, and it will now be apparent from the foregoing to those skilled in the art that changes and modifications may be made without departing from the invention in its broader aspects, and it is the invention, therefore, in the appended claims to cover all such changes and modifications as fall within the true spirit of the invention.

The entire disclosure of Japanese Patent Application No. 2006-56998 filed on March 2, 2006 including specification, claims, drawings and summary are incorporated herein by reference in its entirety.

## Claims

1. A data management system **characterized by** a data transmitter-receiver, a data management server and a mobile communication apparatus, which are mutually connected through a network,
said data transmitter-receiver comprises:
a measurement data receiving means for receiving measurement data transmitted from a data measuring instrument according to a predetermined wireless communication system; and
a measurement information transmitting means for transmitting measurement information based on the measurement data received by said measurement data receiving means to said data management server,
said data management server comprises:
a measurement information processing means that records the measurement information transmitted by said measurement information transmitting means into a data recording unit on the basis of a predetermined management scheme, and transmits a predetermined notification to the mobile communication apparatus having a predetermined relation to the measurement information; and
a recorded data transmitting means for transmitting contents recorded in the data recording unit under a predetermined condition to said mobile communication apparatus on the basis of requesting by the mobile communication apparatus,
said mobile communication apparatus comprises:
a request transmitting means for transmitting a request inputted on the basis of the predetermined notification transmitted by said measurement information processing means to said data management server; and
a contents indicating means for indicating the contents transmitted by said recorded data transmitting means according to a predetermined form.

2. The data management system according to claim 1, wherein the network between said data transmitter-receiver and a telephone carrier side system is a cellular phone communication network or a fixed-line phone network, and the network between the telephone carrier side system and said data management server is a dedicated line network.

3. The data management system according to claim 1 or 2, wherein
said mobile communication apparatus further comprises a response information transmitting means for transmitting response information based on the contents indicated by said contents indicating means to said data transmitter-receiver,
said data transmitter-receiver further comprises a display unit and a display means for displaying the response information transmitted by said response information transmitting means on the display unit.

4. The data management system according to any one of claims 1-3, wherein said data measuring instrument is at least one of a measuring instrument of data that relates to a living body of a target for measurement, a measuring instrument of data that relates to an environment of a target for measurement and a measuring instrument of data that relates to a situation of a target for measurement.

5. A data transmitter-receiver connected with a data management server through a network, **characterized by**:
a measurement data receiving means for receiving measurement data transmitted from a data measuring instrument by a predetermined wireless communication system; and
a measurement information transmitting means for transmitting measurement information based on the measurement data received by said measurement data receiving means to said data management server.

6. The data transmitter-receiver according to claim 5, wherein the network between said data transmitter-receiver and a telephone carrier side system is a cellular phone communication network or a fixed-line phone network, and the network between the telephone carrier side system and said data management server is a dedicated line network.

7. The data transmitter-receiver according to claim 5 or 6, wherein
the measurement information transmitted by said measurement information transmitting means is recorded in a data recording unit by said data management server on the basis of a predetermined management scheme, and a predetermined notification is transmitted to a mobile communication apparatus having a predetermined relation to the measurement information, the mobile communication apparatus connected through the network,
a request inputted to said mobile communication apparatus side on the basis of the predetermined notification is transmitted to said data management server by the mobile communication apparatus,
contents recorded in the data recording unit is transmitted to said mobile communication apparatus by said data management server under a predetermined condition according to the request,
the contents is indicated by using a predetermined form by said mobile communication apparatus, and response information based on the indicated contents is transmitted to said data transmitter-receiver,
the response information is displayed on a display unit of said data transmitter-receiver side.

8. The data transmitter-receiver according to any one of claims 5-7, wherein the data measuring instrument is at least one of a measuring instrument of data that relates to a living body of a target for measurement, a measuring instrument of data that relates to an environment of a target for measurement and a measuring instrument of data that relates to a situation of a target for measurement.

9. A data management server connected with a data transmitter-receiver and a mobile communication apparatus through a network, **characterized by**:
a measurement information processing means that records measurement information based on measurement data transmitted from a data measuring instrument to the data transmitter-receiver according to a predetermined wireless communication system and transmitted from the data transmitter-receiver, on the basis of a predetermined management scheme in a data recording unit, and transmits a predetermined notification to the mobile communication apparatus having a predetermined relation to the measurement information; and
a recorded data transmitting means for transmitting contents recorded in the data recording unit under a predetermined condition to the mobile communication apparatus on the basis of a request by the mobile communication apparatus.

10. The data management server according to claim 9, wherein the network between said data transmitter-receiver and a telephone carrier side system is a cellular phone communication network or a fixed-line phone network, and the network between the telephone carrier side system and said data management server is a dedicated line network.

11. The data management server according to claim 9 or 10, wherein the data measuring instrument is at least one of a measuring instrument of data that relates to a living body of a target for measurement, a measuring instrument of data that relates to an environment of a target for measurement, and a measuring instrument of data that relates to a situation of a target for measurement.

12. A method for data management used by a data transmitter-receiver, a data management server and a mobile communication apparatus which are mutually connected through a network, **characterized by**:
a measurement data receiving step at which the data transmitter-receiver receives measurement data transmitted from a data measuring instrument according to a predetermined wireless communication system;
a measurement information transmitting step at which the data transmitter-receiver transmits measurement information based on the measurement data received in said measurement data receiving step to the data management server;
a measurement information processing step at which the data management server records the measurement information transmitted in said measurement information transmitting step in a data recording unit on the basis of a predetermined management scheme, and transmits a predetermined notification to the mobile communication apparatus having a predetermined relation to the measurement information;
a request transmitting step at which the mobile communication apparatus transmits a request inputted on the basis of the predetermined notification transmitted in said measurement information processing step to the data management server;
a recorded data transmitting step at which the data management server transmits the contents recorded in the data recording unit to the mobile communication apparatus under a predetermined condition, on the basis of the request transmitted in said request transmitting step; and
a contents display step at which the mobile communication apparatus indicates the contents transmitted in said recorded data transmitting step by a predetermined form.
